# EUROPEAN PATENT SPECIFICATION

(11) **EP 1 757 232 B1**
(45) Date of publication and mention of the grant of the patent: **20.05.2009**
(21) Application number: 05018377.1
(22) Date of filing: 24.08.2005
(51) Int. Cl.: A61B 17/00

(54) **Manipulation device particularly for removing the saphenous vein**
Handhabungsvorrichtung insbesondere zum Entfernen der Vena saphena
Dispositif de manipulation notamment pour l'enlèvement de la veine saphene

(43) Date of publication of application: 28.02.2007
(73) Proprietor: Terrini, Alberto, 30174 Venezia (IT)
(72) Inventor: Terrini, Alberto, 30174 Venezia (IT)
(74) Representative: Alagem Modiano, Lara S.

(56) References cited:
- WO-A-99/39632
- FR-A- 2 848 407
- US-A- 5 899 912
- US-A- 6 080 102

## Description

The invention relates to a manipulation device, particularly for removing the saphenous vein.

It is known that removal of the saphenous vein from the leg of a patient is currently performed, according to a widely practiced technique, by manually inserting a separator, which keeps the tissues separated from the vein and forms an operating tunnel, within which suitable instruments are inserted for cutting the collateral veins connected to the saphenous vein, so as to free said vein and allow its extraction by sliding.

The entire operation, which can be preceded by a preoperative step for separating the tissues in contact with the vein, performed by means of an instrument that is similar to the separator mentioned above, is managed by the surgeon by means of an endoscope, which allows to view the situation at the operating tunnel.

U.S. Patent No. 5,899,912 discloses a harvesting apparatus including an extended handle from which a harvesting head depends. The head may include electrocautery means and may be used in conjunction with a visual endoscope integrally formed or provided for within the handle and head. In all the disclosed embodiments, the head defines a central channel for receiving the trunk portion of the blood vessel to be harvested and oriented in a direction parallel to the longitudinal axis of the handle.
U.S. Patent No. 6,080,102 discloses an endoscope excising member comprising a body formed into an elongated tube into which the hard endoscope can be inserted, a large-diameter pipe formed in the body at a position adjacent to the operator's hand, and a transparent leading end connected to the leading end of the body and arranged to cover the leading end section of the hard endoscope.

The insertion, on the part of surgeons, of the instruments designed to cut the collateral veins in the portion of space that lies between the saphenous vein and the overlying separator, and especially the management of said instruments when they must cut the collateral veins after making contact with them, is not always easy, and therefore the aim of the present invention is to devise a manipulation device that facilitates to the greatest possible extent the management of the instruments mentioned above.

The proposed aim is achieved by the subject-matter of claim 1.

Further characteristics and advantages of the present invention will become better apparent from the description of a preferred but not exclusive embodiment of the invention, illustrated by way of non-limiting example in the accompanying drawings, wherein:

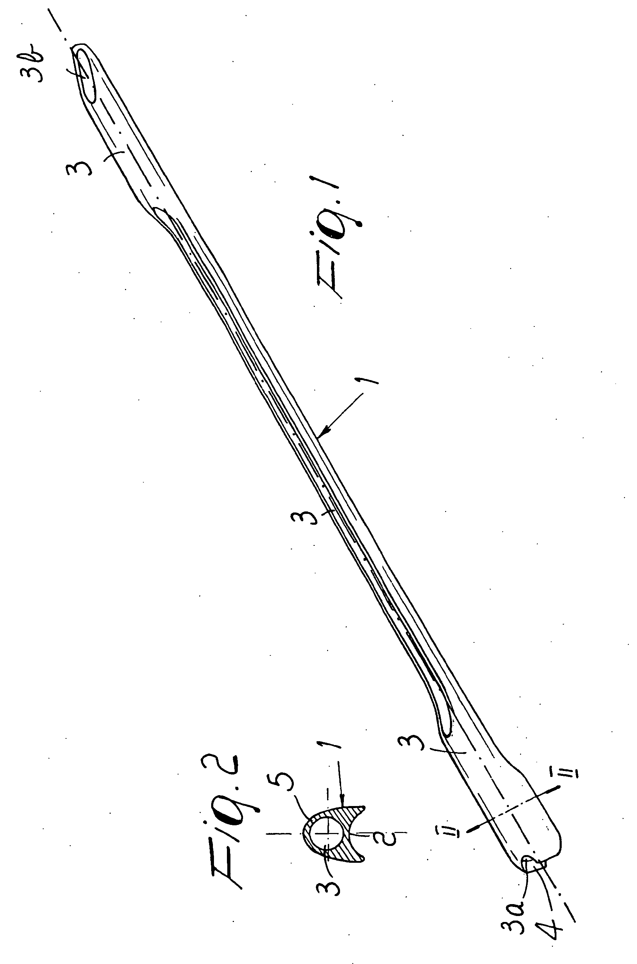

Figure 1 is a perspective view of the invention;
Figure 2 is a sectional view, taken along the line II-II of Figure 1.

With reference to the figures cited above, the reference numeral 1 designates the straight rod, which is shaped so as to form the continuous and slightly arc-like or arched surface 2 at the region that, in use, is intended to face the saphenous vein, so as to ensure optimum conditions in the steps for inserting the device in the leg of the patient, and has such dimensions as to be accommodated in the portion of space that lies between the saphenous vein and the overlying tissue separator inserted beforehand.

The rod 1 comprises internally the longitudinal cavity 3, which is open at its ends 3a and 3b and is designed to accommodate and guide the instruments meant to cut the collateral veins, so as to facilitate to the maximum possible extent its insertion up to the operating region in contact with said veins. The cavity 3 is only on part of its extension completely surrounded by a wall 5.

Furthermore, an important characteristic of the invention consists in that the front end of the rod 1 is shaped according to the arc-like surface 4, whose axis is perpendicular to the axis of the rod 2, so as to surround the collateral vein, which is designed to be cut by the instrument that protrudes from the end 3a of the cavity 3 and thus provide optimum conditions in the cutting operation.

The described invention is susceptible of numerous modifications and variations, all of which are within the scope of the inventive concept as defined in the appended claims; furthermore, all the details may be replaced with other technically equivalent elements.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A manipulation device, particularly for removing the saphenous vein, comprising a straight rod (1) having such dimensions so as to be accommodated in the portion of space that lies between the saphenous vein and an overlying tissue separator, which is inserted beforehand in the leg of the patient and forming a continuous and slightly arc-like surface (2) at a region that faces the saphenous vein **characterized in that** the straight rod (1) has internally a longitudinal cavity (3), which is open at its ends (3a,3b) and being shaped, at its front end (3a), so as to form an arc-like surface (4) with an axis that is perpendicular to the axis of the rod (1) and **in that** the cavity (3) provided within the rod (1) is adapted to allow the passage of instruments meant to cut the collateral veins and is completely surrounded by a wall (5) in proximity of its open ends (3a,3b) and is only partly surrounded by said wall (5) between the open ends (3a, 3b).

## Patentansprüche

1. Handhabungsvorrichtung, insbesondere zum Entfernen der Saphena-Vene, die einen geradlinigen Stab (1) umfasst, der Abmessungen hat, um in dem Raumabschnitt aufgenommen zu werden, der zwischen der Saphena-Vene und einer darüber liegenden Gewebetrenneinrichtung liegt, die im Voraus in das Bein des Patienten eingesetzt worden ist, und in einem der Saphena-Vene zugewandten Bereich eine ununterbrochene und leicht bogenförmige Oberfläche (2) bildet, **dadurch gekennzeichnet, dass** der geradlinige Stab (1) innen einen longitudinalen Hohlraum (3) besitzt, der an seinen Enden (3a, 3b) offen ist und an seinem vorderen Ende (3a) so geformt ist, dass er eine bogenförmige Oberfläche (4) mit einer Achse bildet, die zu der Achse des Stabs (1) senkrecht ist, und dass der Hohlraum (3), der in dem Stab (1) vorgesehen ist, dazu ausgelegt ist, den Durchgang von Instrumenten zuzulassen, mit denen die kolateralen Venen geschnitten werden sollen, und in der Umgebung seiner offenen Enden (3a, 3b) vollständig von einer Wand (5) umgeben ist und zwischen den offenen Enden (3a, 3b) nur teilweise von der Wand (5) umgeben ist.

## Revendications

1. Dispositif de manipulation, en particulier pour retirer la veine saphène, comprenant une tige droite (1) ayant des dimensions telles qu'elle puisse être reçue dans la partie d'espace qui se trouve entre la veine saphène et un séparateur de tissus sus-jacent, qui est inséré au préalable dans la jambe du patient, et formant une surface continue et légèrement en forme d'arc (2) dans une région qui est dirigée vers la veine saphène, **caractérisé en ce que** la tige droite (1) comporte intérieurement une cavité longitudinale (3), qui est ouverte à ses extrémités (3a, 3b) et qui est conformée, à son extrémité avant (3a), de façon à former une surface en forme d'arc (4) avec un axe qui est perpendiculaire à l'axe de la tige (1) et **en ce que** la cavité (3) réalisée à l'intérieur de la tige (1) est adaptée pour permettre le passage d'instruments conçus pour couper les veines collatérales et est complètement entourée par une paroi (5) à proximité de ses extrémités ouvertes (3a, 3b) et n'est que partiellement entourée par ladite paroi (5) entre les extrémités ouvertes (3a, 3b).
